# EUROPEAN PATENT SPECIFICATION

(11) **EP 0 689 601 B1**
(45) Date of publication and mention of the grant of the patent: **04.10.2006**
(21) Application number: 94909777.8
(22) Date of filing: 22.02.1994
(51) Int. Cl.: C12N 15/86, C12N 5/10, A61K 39/00, A61K 48/00

(54) **PRODUCTION OF HIGH TITER HELPER-FREE RETROVIRUSES BY TRANSIENT TRANSFECTION**
HERSTELLUNG VON HELFER-FREIEN RETROVIREN MIT HOHEM TITER MITTELS TRANSIENTER TRANSFEKTION
PRODUCTION DE RETROVIRUSES SANS AUXILIAIRE A TITRE ELEVE, PAR TRANSFECTION TRANSITOIRE

(30) Priority: 22.02.1993 US 23909
(43) Date of publication of application: 03.01.1996
(60) Divisional application: 05076602.1
(73) Proprietor: THE ROCKEFELLER UNIVERSITY, New York, NY 10021 (US)
(72) Inventor: PEAR, Warren, S., New York, NY 10021 (US); NOLAN, Garry, P., Portola Valley, CA 94028 (US); SCOTT, Martin, L., New York, NY 10021 (US); BALTIMORE, David, New York, NY 10011 (US)
(74) Representative: Mercer, Christopher Paul
(86) International application number: PCT/US1994/001983
(87) International publication number: WO 1994/019478

(56) References cited:
- EP-A- 0 854 193
- WO-A-90/02806
- WO-A-92/05266
- WO-A-92/10563
- WO-A-92/10564
- PROCEEDINGS OF THE NATIONAL ACADEMY OF SICENCES vol. 85 , 1988 , WASHINGTON D.C.USA pages 6460 - 6464 DANOS ET AL 'SAFE AND EFFICIENT GENERATION OF RECOMBINANT RETROVIRUSES WITH AMPHOTROPIC AND ECOTROPIC HOST RANGES' cited in the application
- MOLECULAR AND CELLULAR BIOLOGY vol. 7, no. 1 , 1987 , WASHINGTON D.C.,USA pages 379 - 387 DUBRIDGE ET AL 'ANALYSIS OF MUTATION IN HUMAN CELLS BY USING AN EPSTEIN-BARR VIRUS SHUTTLE SYSTEM' cited in the application
- CANCER RESEARCH vol. 49, no. 19 , PHILADELPHIA,USA pages 5429 - 5434 LYNCH ET AL 'IMMORTALIZATION OF PRIMARY BABY RAT KIDNEY CELLS BY RETROVIRAL MEDIATED GENE TRANSFER OF ADENOVIRUS E1A GENES'
- JOURNAL OF GENERAL VIROLOGY vol. 36 , 1977 , COLCHESTER,ENGLAND pages 59 - 72 GRAHAM ET AL 'CHARATERISTICS OF A HUMAN CELL LINE TRANSFORMED BY DNA FROM HUMAN ADENOVIRUS TYPE 5' cited in the application
- BIOLOGICAL ABSTRACTS, xol. 95, no. 3 1993, Philadelphia, PA, US; abstract no. 27884, BROWN ET AL 'ASSESSMENT OF RETROVIRUS-EXPRESSED NUCLEOPROTEIN AS A VACCINE AGAINST LETHAL INFLUENZA VIRUS INFECTIONS OF CHICKENS' & AVIAN DIS. vol. 36, no. 3 , 1992 pages 515 - 520
- PROCEEDINGS OF THE NATIONAL ACADEMY OF SCIENCES,USA vol. 90 , September 1993 , WASHINGTON D.C.,USA pages 8392 - 8396 PEAR ET AL 'PRODUCTION OF HIGH-TITER HELPER-FREE RETROVIRUSES BY TRANSIENT TRANSFECTION'
- MILLER ET AL: 'IMPROVED RETROVIRAL VECTORS FOR GENE TRANSFER AND EXPRESSION' BIOTECHNIQUES vol. 7, no. 9, 01 October 1989, pages 980 - 990

## Description

### TECHNICAL FIELD OF THE INVENTION

The present invention relates generally to the field of molecular biology and, more particularly to the preparation of recombinant retroviral vectors for use in a variety of contexts, including both diagnostic and therapeutic applications, and for research purposes.

### BACKGROUND OF THE INVENTION

Efficient gene transfer is a prerequisite for successful gene therapy. Retroviral vectors are regarded as the most promising vehicles to efficiently introduce selected genes into a wide range of target cells, and have been successfully used in human gene therapy (reviewed in Anderson, 1992). Among the attractive features of retroviral vectors are their abilities to transfer genes efficiently into cells which are difficult to transfect by other methods, to infect hematopoietic stem cells, and to transfer genes in the absence of replication-competent virus.

Present methods for creating high-titer, helper-free retroviral stocks employ the creation of a stable producer cell line that expresses a selected vector (reviewed in Miller, 1990). Producer lines are made by introducing the retroviral vector into a packaging cell line, that synthesizes, in trans, all the proteins required for viral assembly. It is then necessary to select among many individual clones for one clone that express the retroviral vector at high titer, since virus production by the original pool of transfected clones is low, on average. Selection takes at least one month and may be laborious and expensive. In addition, prolonged high expression of the retroviral mRNA may be detrimental to the producer cell line, causing titers to drop with prolonged cultivation.

To be useful in gene therapy, and many other settings, the titer of the virus made by the producer line must be about 10⁶ or greater. For each construct, it is often necessary to test many clonal cell lines to find one that stably produces high titer recombinant virus. Such a procedure takes an average of two months, is labor-intensive, and limits the number of constructs that one can produce. In addition, recombinant constructs often express products that over the long term can be detrimental to the producer line, which leads to a drop in titer as the line is carried. Due to this latter problem, it is sometimes not possible to get high titer for certain constructs.

For example, in attempting to create high titer *v-abl* or P210^{*bcr*/*abl*} helper-free retroviruses, it was found that only very rare clones produced virus at titers greater than 10⁶/ml and that these clones usually lost their ability to produce high titer virus during cultivation. This phenomenon was also observed in retroviral transduction of *rel*-related transcription factors.

A need therefore exists to develop a method for the reliable preparation of viral stocks at high titer that is rapid and efficient in its operation and consistent in its results.

### SUMMARY OF THE INVENTION

In accordance with the present invention, a method is disclosed for the production of high-titer helper-free viral stocks of infectious viral constructs containing a genetic element of interest. The method comprises the preparation of a cell line capable of preparing such viral stocks, introducing a viral construct containing the genetic element of interest into the cell line by transient transfection, and thereafter harvesting the viral stocks prepared thereby. The viral stocks may comprise retroviral stocks and the viral constructs may comprise retroviral constructs The cell line useful in the method may be derived from the Ad5-transformed human embryonic kidney 293 cell line, and in particular, the clonal cell lines prepared herein and named 293T/17 and ANJOU 65. More particularly, the cell line useful in the invention comprises the clonal packaging cell line named BOSC 23 also prepared herein. All three cell lines were deposited on February 12, 1993 with the American Type Culture Collection in accordance with the provisions of the Budapest Treaty,: the cell line 293T/17 has been assigned ATCC Accession No. CRL 11268; ANJOU 65 has been assigned Accession No. CRL 11269; and BOSC 23 has been assigned ATCC Accession No. CRL 11270.

The invention also extends to the new cell lines described above that facilitate the production of high titer helper-free stocks of infectious viral constructs, such as retroviral vectors, in a relatively short period of time following transfection. As stated above, the first cell line prepared in accordance with the invention comprises the 293T/17 cell line, and is derived from Ad5-transformed human embryonic kidney cells known as 293 cells. This cell line is capable of producing high titer retroviral stocks in the presence of helper virus, and may be modified by transfection thereinto of the packaging functions (e.g. by the sequential transfection into 293T/17 cells of the constructs pCRIP^{env-} and pCRIP^{gag-2}) without the development of replication competence.

The ANJOU 65 cell line is derived from the 293T/17 cell line and contains a part of the packaging function necessary for the cell line to serve as a producer cell line in accordance with the present invention. This cell line exhibits high reverse transcriptase activity and likewise produces high titer virus upon transient transfection of a retroviral vector plus helper virus. ANJOU 65 cells require only the introduction of an envelope expression element to serve as a complete packaging cell line. The ANJOU 65 cell line can be used to produce a variety of retroviral and viral constructs, such as amphotropic and pseudotyped retroviruses, including constructs suitable for the infection of human and other cells, thereby facilitating gene therapy.

Further, the invention extends to an ecotropic packaging cell line named herein BOSC 23, and to an amphotropic packaging cell line named CAK 8, both of which cell lines produce high titer helper-free retrovirus. More particularly, both the BOSC 23 and the CAK 8 producer cell lines are much more transfectable than the currently employed packaging cell lines. By using either BOSC 23 or CAK 8 packaging cells and optimizing the CaPO₄ transfection procedure, retroviral titers in excess of 10⁶ infectious particles per milliliter of supernatant can be achieved within 48-72 hours following transfection. The retroviruses produced are helper-free and can infect early hematopoietic progenitors. This transient system thus circumvents the time consuming step of selecting high-titer producer lines. Moreover, titers in excess of 10⁶ may be achieved, as illustrated herein, using retroviral vectors containing genes thought to be "toxic" to stable producer lines. These include the *abl* and *rel*-related genes described above. It is likely that the shortened time for producing retrovirus in the present transient system minimizes any detrimental effects to the producer cells, allowing for high titer production.

Also described herein is a method for the preparation of the clonal producer cell lines by first identifying a cell line, such as the 293 cell line, that exhibits the potential for retroviral production, and introducing into the cell line so selected, constructs that contain the packaging functions needed for the cell line to produce said retroviral constructs. Preferably, such functions are disposed in separate plasmids that are introduced sequentially, to avoid the preparation of replication competent helper virus, so that the constructs prepared by the producing method of the present invention are helper-free. Also, the method for the preparation of the producer cell line preferably proceeds in the presence of a bacterial antibiotic selection medium, as this has been found to facilitate the preparation of the producer cell line having the desired capabilities.

The preparation of high-titer helper-free retrovirus by means of the producer cell lines and corresponding method described herein is useful in a wide variety of diagnostic and therapeutic contexts. Thus, for example, the present invention extends to the preparation of retroviruses expressing particular genetic elements that in turn may be used in direct introduction via gene therapy, for use in gene replacement, complementation of genetic defects, chemotherapy, and the treatment of infectious disorders such as HIV.

The application also discloses viral stocks prepared by the present method and/or by means of the cell lines of the invention. Likewise, cultured cells infected with at least one infectious viral construct derived from the present viral stock are also contemplated herein. Such cultured cells may be prepared for both diagnostic and therapeutic uses. A diagnostic use contemplates the preparation of cellular models for the study of diseases and other states where genetic alteration or dysfunction is present. The cellular models may be prepared by the infection of the cells intended for use in the cellular model with a retroviral vector prepared in accordance with the present invention, that introduces particular genetic material reflecting such genetic alteration of interest. Moreover, in an alternate embodiment of the present invention, multiple retroviral vectors may be introduced to the same cell so as to develop a cellular model reflecting multiple genetic alterations. This would permit the researcher to study the particular condition or conditions resulting from such multiple defects, and moreover, to assess the individual interactions as between the respective genetic alterations. Also, the speed and versatility of the present method facilitates the evaluation of a variety of viral constructs and their effects on cell physiology.

The application further describes a method for the production of proteins from cell types of interest. Specifically, a variety of cell types may be caused to produce modified protein by the introduction thereinto of genetic material in viral stocks prepared by the present invention. Exemplary cell types include explanted patient tissue, animal tissue and cell lines. The present method facilitates the infection of particular cell types to achieve desired tissue-specific modifications, such as protein glycosylation. The production of protein would take place by the infection of target tissue or cells with an infectious viral construct prepared by the present invention, after which the infected target cells would produce the relevant protein of interest containing such modifications as might be expected or predicted from the introduction of the genetic element borne by the particular viral construct.

Also, the large scale production of retroviruses in accordance with the present application facilitates the large scale manufacture of specifically-modified protein as well as preparation of vaccines to accomplish immunity to particular antigens in hosts.

A further application as described herein relates to the isolation of genes from cDNA libraries and the corresponding identification of the function thereof. For example, the products of an entire cDNA library may be expressed by cloning into the retroviral vectors, then transfected into the cell line of the present invention. The large scale production of the retroviral products makes possible the infection of a target population followed by the selection of a particular product according to criteria of interest.

The present invention extends further to the preparation of viruses other than the helper-free ecotropic retroviruses represented by the transient transfection of constructs into the BOSC 23 cell line. More particularly, the ANJOU 65 cell line likewise prepared in accordance with the present invention may be used to prepare amphotropic retroviruses by transfection with the appropriate envelope expressing construct. Also, the present invention extends to the production of helper-free amphotropic retroviruses by transfection of the CAK 8 cell line, as prepared and described herein. In addition, the envelope expressing construct used in the preparation of pseudotyped retroviruses may be the G glycoprotein of vesicular stomatitis virus (VSV), as discussed in Bums, et al., 1993, PNAS USA *90*:8033-8037; and as reviewed in Hopkins, 1993, PNAS USA *90*:8758-8761. The viruses thus prepared are particularly well-suited for the infection of human cell lines as well as the cell lines of other species. Alternative viruses and potential gene therapy vectors, such as adenovirus and herpes viruses, may also be prepared by the techniques disclosed herein.

The present invention also extends to suitable kits for the corresponding assay of the cells having been modified by the present invention. Accordingly, cells containing the genetically engineered constructs introduced in accordance with the present invention may be rapidly assayed to determine gene function and the effects of protein production. Specifically, such kits may be applied to the analysis of gene reporter and inducer constructs, the rapid analysis of mutant constructs and their corresponding biologic function, and the production of biologically-relevant protein in a research setting. Such kits which recapitulate the applications recited earlier for the present invention and contemplate materials including the cell lines have been prepared and may be used in accordance herewith.

Accordingly, the present invention provides the clonal cell line 293T/17 having ATCC Accession No. CRL 11268, the clonal cell line ANJOU 65 having ATCC Accession No. CRL 11269, the clonal cell line BOSC 23 having ATCC Accession No. CRL 11270, and the clonal cell line CAK 8 having ATCC Accession No. CRL11554.
It is a still further object of the present invention to prepare and use assay kits for the analysis of genetically engineered constructs in cells by means of the present invention.

Other objects and advantages will become apparent to those skilled in the art from a review of the ensuing description which proceeds with reference to the following illustrative drawings.

### BRIEF DESCRIPTION OF THE DRAWINGS

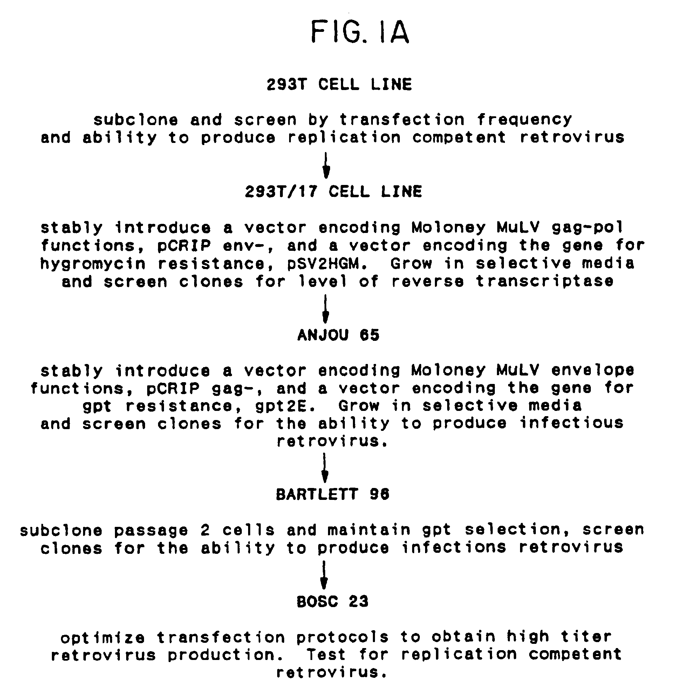

FIGURE 1A is a flow diagram briefly reciting the protocol for the preparation of the BOSC 23 ecotropic producer cell line of the present invention.

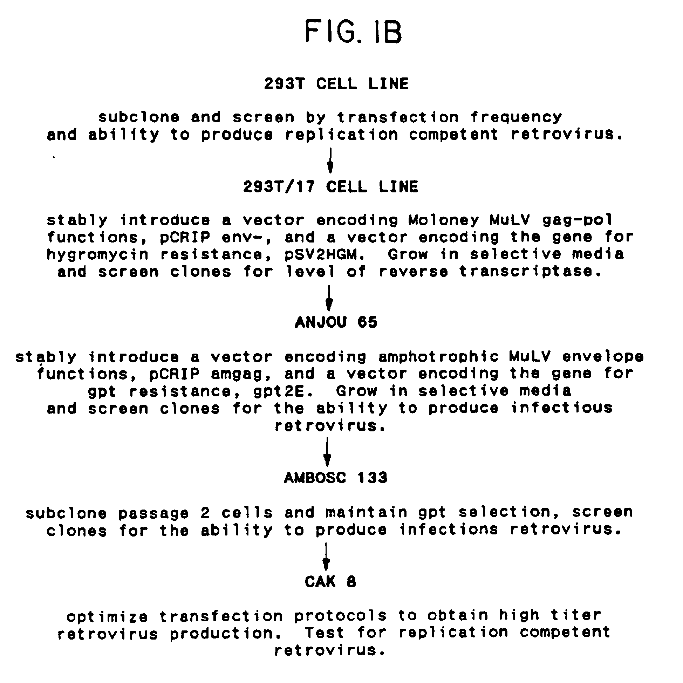

FIGURE 1B is a flow diagram reviewing the protocol for the preparation of the CAK 8 amphotropic producer cell line of the present invention.

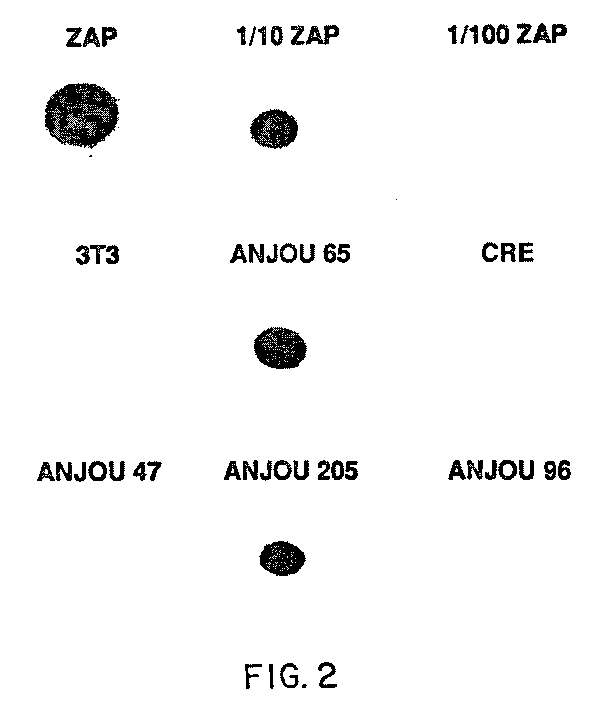

FIGURE 2 depicts the results of RT assays of supernatants from clones of ANJOU cells transfected with pCRIP^{env-}. Individual clones resistant to hygromycin were isolated, and the supernatant was assayed for RT on an exogenous template (Goff et al., 1981). Positive controls are: supernatant from 293T/17 cells transfected with pZAP (wild type Moloney virus, Shoemaker et al., 1981) and supernatant from CRE cells (Danos and Mulligan, 1988). The pZAP transfected supernatants were diluted 1:10 and 1:100 as indicated. NIH 3T3 supernatant is included as a negative control.

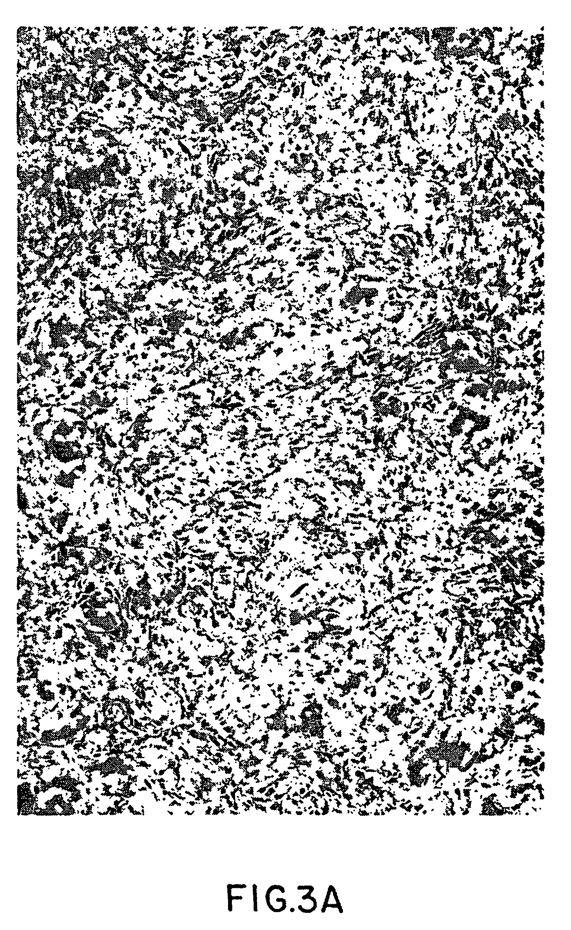

FIGURE 3A shows that pGD^{*v-abl*} virus infection transforms nearly all 3T3 cells. NIH 3T3 cells (5 x 10⁵ cells plated the night before infection) were infected with 1 ml of the 48 hour supernatant from pGD^{*v-abl*} transfected BOSC 23 cells. The corresponding neo titer was 2.9 x 10⁶/ml.

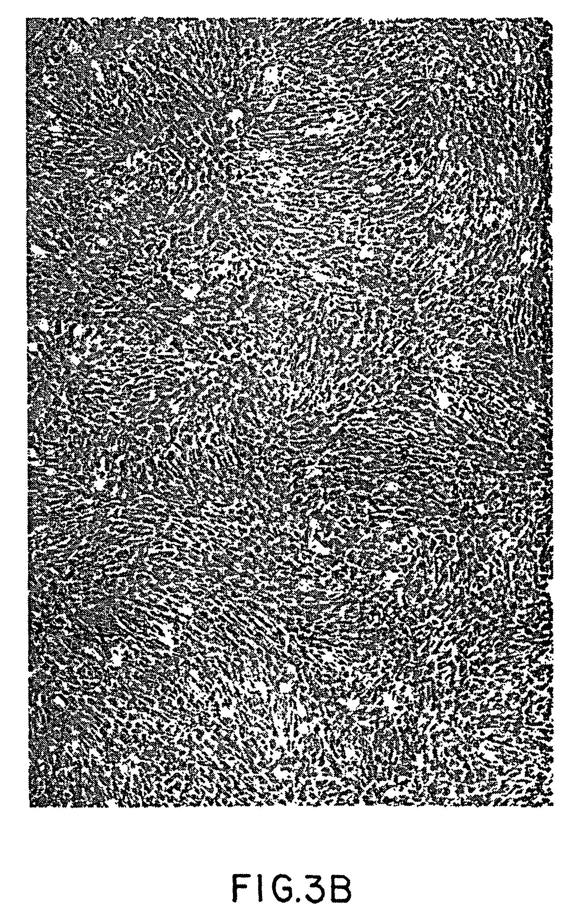

FIGURE 3B depicts the negative control which was pGD infected 3T3 cells (pGD titer was 3.9 x 10⁶/ml). Photographs were taken 48 hours after infection.

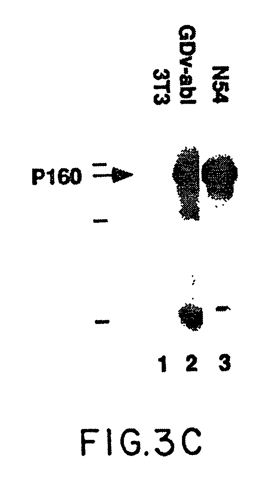

FIGURE 3C depicts kinase activity of fibroblasts infected with a v-*abl* retroviral vector. 3 *µ*g of pGD^{*v*-*abl*} was transfected into BOSC 23 cells in the presence of 25 *µ*M chloroquine as described in the text. 48 hours post-transfection, 1 ml of the supernatant was used to infect NIH 3T3 cells. 48 hours post-infection, the cells were lysed and assayed *in vitro* abl kinase activity (Konopka and Witte, 1985). The immunoprecipitating antibody was pEX-4 (Konopka and Witte, 1985). Prior to the immunoprecipitation, there was approximately half as much protein in the positive control, N54, as in the 3T3 negative control and pGD^{*v-abl*} infected cells. Samples were electrophoresed on a 7.5% SDS-polyacrylamide gel and the phosphorylated proteins were detected by autoradiography for 12 hours with an intensifying screen.

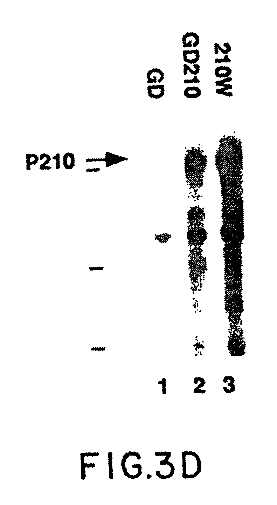

FIGURE 3D depicts kinase activity from fibroblasts infected with a p210^{*bcr*/*abl*} retroviral vector. 3 µg of pGD210^{*bcr*/*abl*} was transfected into BOSC 23 cells in the presence of 25 µM chloroquine as described in the text. 48 hours post-transfection, 1 ml of the supernatant was used to infect NIH 3T3 cells. 48 hours post-infection, the cells were lysed and assayed *in vitro* for *abl* kinase activity (Konopka and Witte, 1985). The immunoprecipitating antibody was pEX-4 (Konopka and Witte, 1985). Prior to the immunoprecipitation, there were approximately equal amounts of protein in the positive control, 210W, as in the pGD-infected negative control and the pGD210^{*bcr*/*abl*} infected cells. Samples were electrophoresed on a 7.5% SDS-polyacrylamide gel and the phosphorylated proteins were detected by autoradiography for 12 hours with an intensifying screen.

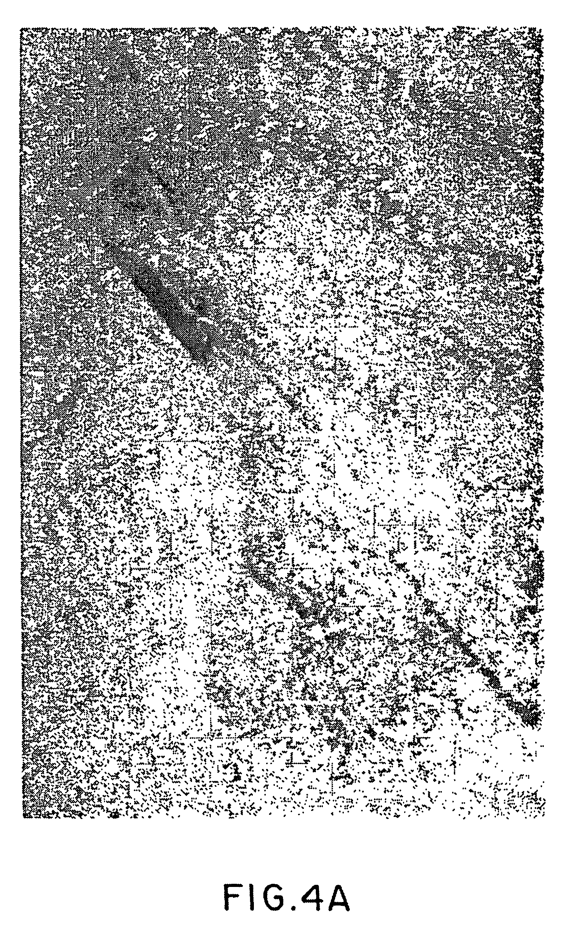

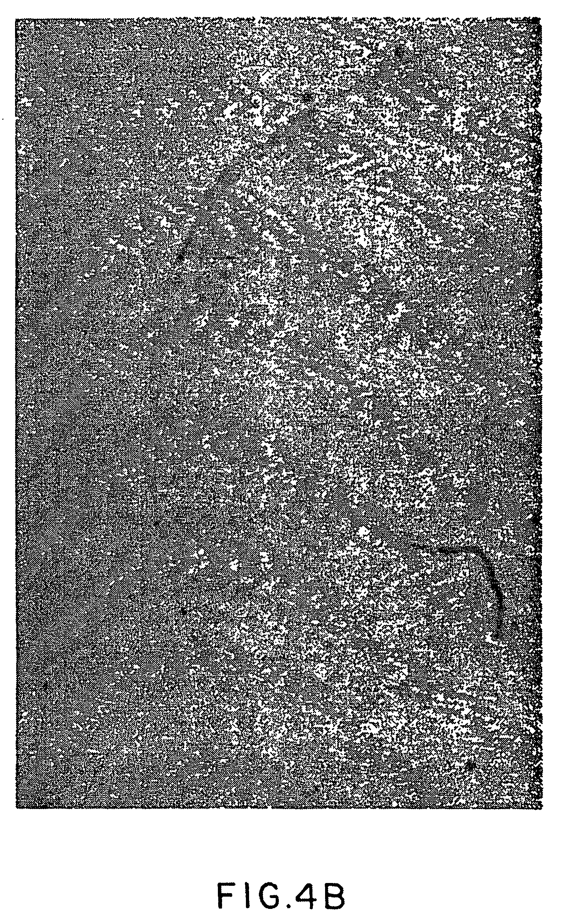

FIGURES 4A and 4B depict the results of histochemical staining of spleens from mice receiving (a) MFG-lacZ -infected bone marrow and (b) pGD infected bone marrow. The mouse transplanted with MFG-lacZ bone marrow received 900 cGy and 5 x 10⁴ bone marrow cells. The mouse transplanted with pGD bone marrow received 850 cGy and 5 x 10⁴ bone marrow cells. (Staining was also negative for the pGD-transplanted mouse who received 850 cGy and 1 x 10⁵ bone marrow cells.) The spleens were fixed and stained and cryostat sections were cut as described in Materials and Methods. Magnification is 10X.

### DETAILED DESCRIPTION

In accordance with the present invention, conventional molecular biology, microbiology, and recombinant DNA techniques may be utilized which are within the level of skill in the art. Such techniques are explained fully in the literature. See, e.g., Sambrook, et al., "Molecular Cloning: A Laboratory Manual" (1989); Ausubel, et al., *Current Protocols in Molecular Biology,* (John Wiley and Sons, Inc., New York, 1989); "DNA Cloning: A Practical Approach," Volumes I and II (D.N. Glover ed. 1985); "Oligonucleotide Synthesis" (M.J. Gait ed. 1984); "Nucleic Acid Hybridization" (B.B. Hames & S.J. Higgins ed 1985); "Transcription And Translation" (B.D. Hames & S.J. Higgins eds. 1984); "Animal Cell Culture" (R.I. Freshney ed 1986); "Immobilized cells And Enzymes" (IRL Press, 1986); B. Perbal, "A Practical Guide to Molecular Cloning" (1984).

A "replicon" is any genetic element (e.g., plasmid, chromosome, virus) that functions as an autonomous unit of DNA replication *in vivo;* i.e., capable of replication under its own control.

A "vector" is a replicon, such as plasmid, phage or cosmid, to which another DNA segment may be attached so as to bring about the replication of the attached segment.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA).

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background.

Within the promoter sequence will be found a transcription initiation site (conveniently defined for example, by mapping with nuclease S1), as well as protein binding domains (consensus sequences) responsible for the binding of RNA polymerase. Eukaryotic promoters will often, but not always, contain "TATA" boxes and "CAT" boxes.

A coding sequence is "under the control" of transcriptional and translational control sequences in a cell when RNA polymerase transcribes the coding sequence into mRNA, which is then translated into the protein encoded by the coding sequence.

A "signal sequence" can be included before the coding sequence. This sequence encodes a signal peptide, N-terminal to the polypeptide, that communicates to the host cell to direct the polypeptide to the cell surface or secrete the polypeptide into the media; this signal peptide is clipped off by the host cell before the protein leaves the cell. Signal sequences can be found associated with a variety of proteins native to prokaryotes and eukaryotes. For instance, alpha-factor, a native yeast protein, is secreted from yeast, and its signal sequence can be attached to heterologous proteins to be secreted into the media (See U.S. Patent 4,546,082, EPO 0 116 201, publication date 12 January 1983; U.S. Patent Application Serial No. 522,909, filed 12 August 1983). Further, the alpha-factor leader and its analogs have been found to secrete heterologous proteins from a variety of yeast, such as Saccharomyces and Kluyveromyces, (EPO 88312306.9 filed 23 December 1988; U.S. Patent Application Serial No. 139,682, filed 30 December 1987, and EPO Publication No. 0 301 669, publication date 1 February 1989).

A cell has been "transformed" by exogenous or heterologous DNA when such DNA has been introduced inside the cell. The transforming DNA may or may not be integrated (covalently linked) into chromosomal DNA making up the genome of the cell. In prokaryotes, yeast, and mammalian cells for example, the transforming DNA may be maintained on an episomal element such as a plasmid.

With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth *in vitro* for many generations.

A "retrovirus" is a virus that contains genomic RNA that directs the formation of a DNA molecule, that in turn acts as the template for the production of the mRNA for the construction of new viruses. A "retroviral vector/construct" is an infectious retrovirus that is used to introduce a non-viral gene into mitotic cells both *in vitro* and *in vivo*. More generally, a virus is a cellular parasitic particle comprising nucleic acid (either RNA or DNA) contained within an outer shell of protein known as the capsid. The infectious virus particle is also known as a "virion." When used herein, the term "virus" is accordingly intended to include retroviruses within its scope.

A "viral stock" is the virus-containing supernatant prepared from cells that have been transfected or infected with a viral vector or construct. Such cells include those described and disclosed herein. The term "retroviral stock" is used when referring to infectious retroviral particles prepared in this fashion.

"Replication competent" viral constructs are constructs that retain all of the genes encoding the virion structural proteins, as well as the cis-acting viral elements necessary for transmission. Virus is considered "replication incompetent" when some or all of the genes encoding virion structural proteins are deleted or absent.

"Helper virus" is a replication-competent virus that is sometimes present in stocks of replication-incompetent virus. In the instance of retroviral production as by the practice of the present method, the presence of helper virus is a contaminant that is desirably avoided. For example, if animal infections are being done, helper virus could lead to the development of unwanted pathological conditions in the host.

An "ecotropic" retrovirus is a virus bearing a murine viral envelope glycoprotein that binds to the "ecotropic receptor" found only on rat and mouse cells. The ecotropic glycoprotein generally does not allow infection of human cells. By contrast, an "amphotropic" retrovirus is a retrovirus, including a murine virus, that contains the *env* genes of the amphotropic class of glycoproteins, and that can bind to a broad range of hosts, including rodent, mouse, human, chicken, dog, cat and mink.

The terms "genetic material" and "genetic element(s) of interest" as used herein refer broadly to nucleic acids (e.g. RNA and DNA), and more particularly, to such materials as may be desirably introduced into a target cell by incorporation within a viral construct prepared in accordance with the present invention. Particular "genetic materials/elements of interest" include the following: antisense nucleic acid; DNA; mRNA; ribosomal RNA; recombinant RNA; small nuclear RNAs; nucleotides that encode a member selected from the group consisting of proteins, fragments of proteins, antisense nucleic acid, DNA, mRNA, ribosomal RNA, recombinant RNA, and small nuclear RNAs; fragments of any of the foregoing; and combinations thereof.

A composition comprising "A" (where "A" is a single protein, DNA molecule, vector, etc.) is substantially free of "B" (where "B" comprises one or more contaminating proteins, DNA molecules, vectors, etc.) when at least about 75% by weight of the proteins, DNA, or vectors (depending on the category of species to which A and B belong) in the composition is "A". Preferably, "A" comprises at least about 90% by weight of the A+B species in the composition, most preferably at least about 99% by weight. It is also preferred that a composition, which is substantially free of contamination, contain only a single molecular weight species having the activity or characteristic of the species of interest.

In its primary aspect, the present invention concerns the preparation and use of specially engineered retroviral producer cell lines derived from the Ad5-transformed human embryonic cell line 293, to serve as a packaging cell line for the preparation of retroviral stocks containing a genetic element(s) of interest, that are high-titer and are free of helper virus. The present method accordingly comprises:
A. preparing a cell line capable of preparing said viral stocks;
B. transiently transfecting a viral construct containing said genetic element of interest into said cell line; and
C. harvesting the viral stocks prepared thereby.

More particularly, the invention extends to the clonal cell lines described earlier herein, named 293T/17, ANJOU 65, BOSC 23, and CAK 8, and to cell lines derivative therefrom. The clonal cell lines of the invention facilitate the preparation of large stocks of desired retrovirus by the present method, which is based on transient transfection. All of the cell lines disclosed and claimed herein have been deposited with the American Type Culture Collection (ATCC). The first three cell lines have been assigned a date of deposit of February 12, 1993, and the following Accession Numbers. 293T/17 - Accession No. CRL 11268; ANJOU 65 - Accession No. CRL 11269: BOSC 23 - Accession No. CRL 11270. CAK 8 has been deposited with the ATCC on February 22, 1994 and has been assigned Accession No.

The present invention extends to the method for the preparation of the clonal cell lines by the selection and isolation of a quantity of suitable cells such as the 293 cells, followed by the introduction into such cells of constructs such as plasmids, that render the cells capable of producing virus. In particular, the plasmids contain the elements that facilitate the packaging of the virus by the replication of the viral sequence followed by the encapsidation of the virus by the envelope function.

One of the aspects of the present method comprises the sequential introduction of the constructs that enable the cell line to replicate and package the virus, as this has been found to confer the desired attributes of the helper virus without enabling such constructs to develop replication competence. Also, the producer cell line is preferably maintained in a bacterial antibiotic selection medium such as the gpt selection medium described later on herein.

The distinguishing characteristics of transient transfection into a highly transfectable helper-producer cell line is that each cell transfected contributes to the production of retroviruses. Thus, in a further embodiment of the invention, each cell may be directed to take up different viruses. Given that 3 x 10⁶ cells transiently transfected at 66% efficiency with 5 *µ*g of DNA (4 x 10¹¹ DNA molecules of length 10 kB) produce up to 10⁷ retrovirus/ml, then each transfected cell produces approximately 0.5 infectious viruses per ml. Thus, this approach will allow for the efficient introduction of a retroviral expression library into target cells.

As mentioned earlier and in further detail later on herein, the invention extends to the viral stocks prepared by the present method, as well as to cultured cells and vaccines prepared from using such stocks. Likewise, genetic aberrations or dysfunctions may be treated by the preparation of a rehabilitative genetic construct within a virus by the present method, followed by the administration of such construct to target cells to treat resident aberrations or dysfunctions. Cultured cells may be infected with multiple and different viral constructs to simulate correspondingly multiple genetic alterations.

In addition, the invention extends to the use of certain of the cell lines that were developed intermediate to the preparation of BOSC 23 and CAK 8, and that possess independent utility in the preparation of alternate vectors of interest. For example, the ANJOU 65 cell line may be used to prepare a producer line for certain alternate viruses, such as amphotropic and pseudotyped retroviruses, that will be useful in the infection of mammalian and other species including humans. Particularly, and as described herein, ANJOU 65 has been used to prepare the amphotropic producer cell line CAK 8, and likewise, the invention contemplates the preparation of a pseudotyped retrovirus such as that including an envelope construct derived from vesicular stomatitis virus (VSV), as discussed in Bums et al. *supra..*

As described earlier herein and above, the present invention offers significant diagnostic and therapeutic applications. The vectors or constructs prepared by the invention may be used in studies of particular animal disease models, for example, β-thalassemia or Gaucher's diseases, that may serve in drug discovery efforts. Likewise, therapeutic protocols may be developed to deliver particular therapeutically active genes to host cells in need thereof by means of the direct *in vivo* introduction of retroviral vectors prepared in accordance with the present invention. The ability to administer such gene therapy finds particular utility in organs such as the bone marrow, whose cells are derived from a population of pluripotential progenitor cells, and the invention extends to a method that involves bone marrow transplantation. Naturally, the invention may find utility in adaptation to other organs where similar strategies are possible. The above is presented by way of illustration and not limitation.

The invention correspondingly contemplates compositions for administration in accordance with the aforedescribed methods, comprising appropriate unit doses of vectors or infectious constructs prepared in accordance with the present invention. Predetermined quantities of a particular retroviral vector or infectious construct may be prepared and administered in accordance with appropriate protocols for such therapy. The exact quantities will vary with the particular therapy and are variable within the discretion of the skilled physician.

The applications of the methods and materials of the invention to both the medical and biotechnical fields are discussed in greater detail below:
**GENE THERAPY:** The invention allows rapid production of high titer helper-free retroviral stocks expressing genetic elements of interest as defined herein. Such genetic elements include those encoding proteins, pieces of proteins, messenger RNAs, ribosomal RNAs, small nuclear RNAs, engineered RNAs and proteins, antisense nucleotides, as well as other genetic material; all of these are under the control of genetic regulatory elements. The shortcomings of previous gene therapy approaches have included the time-consuming creation of stable cell lines (greater than one month is involved) and the difficulty of generating high titer retroviral producer lines. The present method overcomes these two major limitations, and therefore, will greatly facilitate the introduction of recombinant retroviruses into target cells for the purpose of providing medical therapy.
   Production of high titer helper-free retroviral stocks in a very short time (two to three days) by the present method accelerates, and in some cases makes possible, gene therapy technology. Specific steps of gene therapy facilitated by the present invention are: 1) the necessary testing of multiple different genetic constructs in different cell types for their efficacy in specific protein production, their cellular toxicity, and their cell type specificity, 2) customization of retroviruses for specific patient needs, and 3) production of high titer helper-free retroviruses for use in gene replacement, complementation of genetic defects, chemotherapy, and the treatment of infectious disorders such as HIV.
**DISEASE AND DEVELOPMENTAL MODELS:** Another area to which the invention may be applied is the development of models of human and animal disease. As discussed above, limitations of the present methodologies make it difficult to establish models of diseases which result from infection, transformation, or other perturbations in a rare target cell. Use of the invention should facilitate the creation of models for such diseases as chronic myelogenous leukemia, aplastic anemia, HIV, as well as early stages of carcinogenesis. By allowing infection of the same cell with multiple retroviral vectors, our invention may also permit study of diseases which result from multiple genetic alterations. This methodology makes it possible to rapidly test a number of different genetic constructs in order to dissect disease pathogenesis.
   By utilizing a transient transfection method for the production of high titer helper-free retrovirus, the invention will facilitate the introduction of genetic material into multiple different cell types. Such tissue types include explanted patient tissue, animal tissue, and cell lines. The biologic activity of different constructs and their effects on cellular physiology can be rapidly ascertained.
   The invention also makes it possible to study development by using the high titer viruses to mark specific cell types, even when rare. Studying the progeny of cells containing the marker should allow dissection of developmental pathways. Likewise, it should be possible to mark rare cells in a population, such as residual tumor cells following treatment.
**PROTEIN PRODUCTION:** The invention allows specific protein production from cell types of interest, including primary cells and cultured mammalian cell lines. The method comprises the preparation of a viral stock containing the genetic material or element of interest, followed by the introduction of the viral stock into a target cellular colony for the production of the protein in object. The methods presently in use suffer from both the need to make stable cell lines and the potential long-term toxicity to the producing cell of the generated product.
   For pharmaceutical purposes these viruses can be used to infect appropriate cell types. These cells then produce large quantities of the relevant proteins with appropriate tissue-specific modifications. For example, if glycosylation is necessary for the function of a particular protein, the protein can undergo this modification after infection of the appropriate cell type.
   The present invention can also be used to produce retroviral constructs which can be used for immunization. The introduction by viruses of antigenic material for the production of vaccines has long been used to stimulate immunity in vaccine recipients. Retroviral constructs expressing specific antigenic material can be rapidly prepared at high titer using the present method. These viruses can be used as vaccines to induce immunity directly, or, can be used to infect appropriate recipient cells to produce large quantities of specifically-modified protein.
**GENE ISOLATION:** The invention should greatly increase the ability to isolate genes from cDNA libraries and identify their function. Because of the rapid production of viruses with high titer, it should now be possible to express the products of cDNA libraries by cloning them into retroviral vectors which are then transfected into one of the clonal producer cells disclosed herein. These viruses can then infect a target population, and the product is selected by the criteria of interest. For example, genes expressed on the cell surface may be identified by FACS or cell lines mutant in a defective gene may be complemented with a retroviral library from a wild-type tissue source. Similarly, dominant negative and dominant positive mutations of polypeptides may be identified by appropriate selection. Once cells are selected, it is relatively easy to identify the cDNA responsible for the effect since it exists as an integrated provirus of known genetic structure.
**PRODUCTION OF OTHER VIRUSES:** A similar approach to that described herein for producing helper-free ecotropic retroviruses is possible for the production of other types of retroviruses, as well as other types of viruses. Amphotropic and pseudotyped retroviruses can be easily made by transfecting the appropriate envelope expressing construct into ANJOU 65 cells. The preparation and use of the CAK 8 cell line is illustrative of the former. The selection method is facilitated by selecting those clones with high envelope production on FACS (Nolan and Pear, unpublished). These amphotropic and pseudotyped viruses will be essential for infecting human and cell lines of other species.
   In order to decrease the potential for creating replication-competent virus, it may be possible to express the packaging functions from constructs which do not contain homology to the retroviral vectors. Markowitz and Banks have shown that it is possible to produce high titer retroviruses by placing the packaging functions on two different plasmids whose only area of homology outside the 5' LTR was a small region of overlap in the 5' portion of the envelope gene (Markowitz et al., 1988). These vectors contain much less homology to each other than the CRIP vectors which share extensive homology throughout the retroviral genome. It should be possible to further modify the approach of Markowitz and Bank by replacing the 5' LTRs with promoters, such as CMV, which do not share homology with the 5' LTR of the retroviral vector. This should reduce the possibility of recombination resulting in replication competent retrovirus by several orders of magnitude.
   The present invention is considered to extend to the production of high titers of viruses other than retroviruses. These include such potential gene therapy vectors as adenovirus, and herpes viruses. If one is able to build the packaging functions into a 293-derivative cell line, it should be possible to transiently produce a wide variety of high titer helper-free viruses.
**KITS:** The invention also has wide application in other areas of recombinant DNA technology. An important aspect of the technology is the introduction of genetically engineered constructs into cells and the assay of the subsequent effects. A test kit may accordingly be prepared that may be used to measure and assess the activity of genetic material or element(s) of interest on a target cellular population, that comprises one of the cell lines of the invention in combination with reagents for optimal transfection. The present invention greatly improves the introduction of genetic material into mammalian cells. It is envisioned that kits for the rapid assay of gene function and the effects of protein production in mammalian cells can be developed. Specific uses for such kits include 1) analysis of gene reporter and inducer constructs, 2) rapid analysis of mutant constructs and their biologic function, and 3) production of biologically-relevant protein in a research setting. The system has already been used to overexpress proteins in cells in order to determine subcellular localization, and the use of reporter constructs based on retroviruses to assay transcriptional function has already been established.
   The present invention will be better understood from a review of the following illustrative description presenting the details of the cell lines prepared and the constructs introduced and the procedures that were followed in their development and validation. Specific examples will be presented thereafter.

### MATERIALS AND METHODS

**Cell Lines and Plasmids.** 293T cells (DuBridge et al., 1987), originally referred to as 293tsA1609neo, were obtained from S. Haase (Stanford) and grown in Dulbecco's modified Eagle's medium (DMEM) containing 10% (vol/vol) fetal calf serum. Drug selections in transfected 293T cells were done at 400 ng/ml hygromycin (Calbiochem) for hygromycin resistance and 50 ng/ml mycophenolic acid for gpt resistance. Individual clones were isolated by sterile cloning rings and transferred to 24-well plates. The following cell lines, all grown in DMEM containing 10% (vol/vol) calf serum were used: NIH 3T3, BAG (Price et al., 1987), N54 (Jackson and Baltimore, 1989), and 210W (Lugo and Witte, 1989).

The following plasmids were used: pBND 8 (Turner and Cepko, unpublished), a retroviral vector derived from the BAG vector (Price et al., 1987) in which *β-*galactosidase expression is directed by the viral promoter in the LTR and the neo cassette is deleted, pSPUD (Walsh and Cepko, 1988), pCRIP^{env-} (Danos and Mulligan, 1988), pCRIP^{gag-2} (Danos and Mulligan, 1988), pCRIP^{amgag} (Danos and Mulligan, 1988), MFG-lacZ, a retroviral vector containing additional packaging sequences from the gag region (Bender et al., 1987) and expressing *β-*galactosidase from the retroviral LTR (Spain et al., unpublished), MFG-tPA, (similar to MFG-lacZ, but expressing the human tPA gene in place of *β-*galactosidase, (Spain et al., unpublished), pZAP (Shoemaker et al., 1981), pSV2Hgm (Bernard et al., 1985), GPT2E (Jasin and Berg, 1988), pGD (Daley et al., 1990), pGD^{*v-abl*} (Scott et al., 1991), and pGD210^{*bcr*/*abl*} (Daley et al., 1990) are as previously described. All plasmids were grown in *Escherichia coli* DH5α.

**Enzymatic Assays and nucleic acid procedures.** Staining for β-galactosidase activity in intact cells and spleen was done according to MacGregor et al. (MacGregor et al., 1990). The FACS-gal assay was performed as previously described (Nolan et al., 1988). Reverse transcriptase activity was assayed in the culture medium of exponentially growing cells as described by Goff et al. (Goff et al., 1981). The *in vitro abl* kinase assays were performed as described by Konopka et. al. using anti-pEX4 as the immunoprecipitating antibody (Konopka and Witte, 1985). Isolation of DNA and blot analysis were performed by standard procedures (Ausubel et al., 1989).

**Transfections, Infections, and Determination of Viral Titers.** Except where noted, transfection of calcium phosphate/DNA co-precipitates was done on 60 mm dishes where 1.5-2.5 x 10⁶ 293T cells (or derivatives) were plated in 4 mls of DMEM with 10% FCS the night prior to transfection. Chloroquine was added to this media to a final concentration of 25 *µ*M approximately 15-30 minutes prior to adding the DNA/HBS solution. Either 250 ml or 500 ml HBS (pH=7.05) was added to an equal volume of DNA/CaCl₂ solution by bubbling for 10 seconds and the resulting solution was immediately added to the above media, after which the cells were returned to the 37° incubator (5% CO₂) (Heinzel et al., 1988). Twenty four hours after infection, the media was changed to 3 mls of fresh DMEM containing 10%FCS. Twenty four to forty eight hours thereafter, the media was removed and filtered through a 0.45 µm filter or centrifuged at 1500 RPM for 5 minutes in a Sorvall RT6000B centrifuge. In experiments with chloroquine, the media was changed to 10% FCS at 10 hours post-transfection and changed a second time at 24 hours post-transfection. Infections were in a total volume of 3 mls of 10% CS containing 8 *µ*g/ml Polybrene; which was added to 3T3 cells that had been plated at a density of 5 x 10⁵ cells on a 10 cm dish the night prior to infection. Seven mls of media containing 10% CS was added to the dishes after 3 hours and the cells were stained for β-galactosidase activity 48 hours later. Viral titer was determined as the average number of blue cells per 10-25 high power fields (40,000-100,000 total cells) multiplied by the magnification; or when FACS analysis was performed, the percentage of positive cells was multiplied by the total number of cells. G418 selection was performed as above except at 48 hours post-infection, the cells were split 1:10 into selective media, refed every 3 days, and colonies were counted 12 days later. In determining the G418 titer, the number of colonies was divided by 4 to account for 2 cell doublings.

**Helper Virus Assay.** 1 ml of supernatant from transfected BOSC 23 cells was used to infect BAG cells as described above, and the cells were split 1:10 every 3-4 days. To determine the titer of the virus used to infect the BAG cells, 1 ml of the viral supernatant from the transfected BOSC 23 cells was used in parallel to infect 3T3 cells which were assayed for β-galactosidase or G418 resistance. When passage 3, 5, and 10 of the BAG-infected cells had reached approximately 50% confluence, the media was changed, and 24 hours later, 3 mls of this supernatant was filtered (0.45 *µ*M) and used to infect 3T3 cells that were stained for *β-*galactosidase activity 48 hours later. For the 293T/17 experiment, 5 *µ*g of pCRIP^{env-}, pCRIP^{gag-2}, and pBND 8 were co-transfected into 293T/17 cells and 1 ml of viral supernatant was used to infect the BAG cells. The BAG cells were treated as above except that β-galactosidase staining was performed on passage 3 cells only.

**Bone marrow transplantation.** Bone marrow transplant was performed as described previously (Daley et al., 1990). 11 month old female Balb/c mice were recipients and donors were 11 month old male Balb/c mice treated with 2 ng 5-fluorouracil (5-FU) 4 days prior to bone marrow harvest. Cocultivation was done in WEHI-3B conditioned media with BOSC 23 cells which had been transfected with 6 *µ*g MFG-lacZ prior to cocultivation. Non-adherent cells were removed 48 hours later and injected into the lateral tail vein of recipient mice which had received 850 or 900 cGy (2 doses split by 3 hours) at cell concentrations ranging from 2.5 x 10⁴ to 1 x 10⁵ per mouse. Surviving mice were sacrificed at d13 and the spleens were analyzed.

### EXAMPLE 1

The production of high titer retroviral stocks by transient transfection appears to be limited by the transfectability of the producer cells, type of expression constructs, and the copy number of the introduced constructs. Previous studies in this lab had shown that the Ad5-transformed embryonic kidney cell line, 293 (Graham et al., 1977), was significantly more transfectable than NIH 3T3 cells, the cell line from which most producer cell lines have been derived. As a result, it was hypothesized that transient transfection of a producer line based on 293 cells would produce retroviral vectors with higher titers than transient transfection of 3T3-based producer cell lines.

In order to maximize the chances of transiently obtaining high titer retroviral vectors, the system was developed using the 293T line; a 293 subline which contains the SV40 large T antigen (DuBridge et al., 1987). Since 293T contains large T, the copy number and viral titer of constructs containing an SV40 origin of replication should be increased. In addition, vectors based on Moloney murine leukemia virus were chosen, because these vectors were capable of producing titers greater than 10⁶/ml in the best stable producer cell lines (Miller, 1990).

To test the hypothesis that transient transfection of 293 cells could be used to produce high titer retroviral vectors, it was initially investigated whether it was possible to obtain high retroviral vector titers in the presence of helper virus. As a first step, the 293T cell line was cloned by limiting dilution in order to insure that the starting population was uniformly highly transfectable. 14 independent clones were screened by transfecting a replication-defective retroviral construct containing the lacZ gene pBND 8, (Turner and Cepko, unpublished). 48 hours later, cells from each clone were histochemically stained to detect the lacZ gene product. Greater than 50% of the cells from 9 clones and from the parental population expressed β-galactosidase, while, less than 25% of cells from the 5 remaining clones stained positive (data not shown).

To determine which clone gave the highest infectious retroviral titer, 7 of the 9 clones exhibiting high transfectability were assayed for infectious retrovirus. The assay system was to cotransfect equimolar amounts (3 *µ*g) of pBND 8 and a clone of replication competent Moloney MuLV (pZAP (Shoemaker et al., 1981)) onto 5 x 10⁵ cells of the particular 293T subclone which had been plated 16-24 hours previously. At 48-72 hours post-transfection, 1 ml of the viral supernatant was used to infect 3T3 cells; which were stained for β-galactosidase 48 hours after infection. Using this assay, 3 clones gave the highest titer of approximately 1.5 x 10⁶ (data not shown). One of these clones (subsequently referred to as 293T/17) was chosen for further analysis. In subsequent experiments, we were able to obtain a retroviral titer of 4.6 x 10⁶ after transfecting 2 x 10⁶ 293T/17 cells with 7.5 *µ*g of both pBND 8 and pZAP. The results are presented in Table 1, below.

**TABLE 1**

| Retroviral production from BOSC 23 and progenitors cells after transfection | | | | |
|---|---|---|---|---|
| CELL LINE (p=passage number) | CONSTRUCTS | CHLOR^{a} | TITER^{b} | HELPER^{c} |
| 293T/17 | 7.5 µg pBND | - | 4.6 x 10⁶ | + |
| | 7.5 µg pZAP | | | |
| 293T/17 | 5 *µ*g pBND | - | 1.1 x 10⁵ | + |
| | 5 *µ*g pCRIP^{gag-2} | | | |
| | 5 *µ*g pCRIP^{env-} | | | |
| ANJOU 65 | 3.5 *µ*g pBND | - | 1.5 x 10⁵ | |
| | 3.5 *µ*g pCRIP^{gag-2} | | | |
| ANJOU 65 | 2 µg pBND | - | 2 × 10⁵ | |
| | 2 *µ*g pCRIP^{gag-2} | | | |
| | 2 µg pCRIP^{env-} | | | |
| ANJOU 65 | 3.5 µg pBND | - | 0 | |
| BARTLETT 96, p2 | 3 µg pBND | - | 1.6 x 10⁶ | |
| BARTLETT 96, p8 | 3 µg pBND | - | 6 x 10⁵ | |
| BOSC 23, p2 | 3 µg MFG-lacZ | - | 2.9 x 10⁶ | |
| BOSC 2y3, p6 | 3 µg MFG-lacZ | + | 8.6 x 10⁶ | |
| BOSC 23, p12^{d} | 6 µg MFG-lacZ | +^{a} | 1.5 x 10⁷ | |
| BOSC 23, p12^{f} | 3 µg MFG-lacZ | - | 6.5 x 10⁶ | |
| BOSC 23, p6 | 3 µg pGD | + | 3.9 x 10^{6 g} | - |
| BOSC 23, p6 | 3 µg pGD^{v-abl} | + | 2.9 x 10^{6 g} | - |
| BOSC 23, p6 | 3 µg pGD210^{bcr/abl} | + | 1.1 x 10^{6 g} | |
| BOSC 23, p6 | 3 µg MFG-tPA^{h} | + | | - |
| AMPHO 18, p2 | 3 µg MFG-lacZ | + | 0 | |
| AMPHO 133, p2 | 3 µg MFG-lacZ | + | 2.0 x 10⁶ | |
| CAK 8, p3 | 3 µg MFG-lacZ | + | 2.0 x 10⁶ | |

| | | | | |
|---|---|---|---|---|
| NOTES: ^{a}: presence or absence of 25 uM chloroquine as described in Materials and Methods ^{b}: infectious titer per ml (βgal positive cells) ^{c}: presence or absence of replication competent virus as described in Materials and Methods ^{d}: 60% of cells were transfected as assayed by FACS (see Materials and Methods) ^{e}: volume of all transfection reagents was doubled ^{f}: 302% of cells were transfected as assayed by FACS (see Materials and Methods) ^{g}: titers determined by G418 resistance ^{h}: not titered, but MFG-lacZ transfected in parallel had a titer of 7 x 10⁶/ml | | | | |

These results showed that it was possible to obtain high-titer retroviral vectors by transient transfection of 293 cells.

### EXAMPLE 2

**STRATEGY FOR CREATING THE ECOTROPIC HELPER-FREE CELL LINE:** The next step was to build a helper-free producer from the 293T/17 subline. The preparation of an ecotropic cell line is reviewed in Figure 1A. The strategy for creating a helper-free producer cell line was determined to be to stably transfect plasmids containing the Moloney packaging functions into the 293T/17 cell line. To accomplish this, a set of plasmids, pCRIP^{env-} and pCRIP^{gag-2}, was used which placed the Moloney packaging functions onto 2 plasmids (Danos and Mulligan, 1988). pCRIP^{env-} contains a mutation in the envelope region and expresses both gag and pol products. pCRIP^{gag-2} contains mutations in the gag region, and only expresses the ecotropic envelope (Danos and Mulligan, 1988). In addition, the retrovirus RNA packaging site was deleted and the 3' LTR was replaced by an SV40 polyA site in both plasmids (Danos and Mulligan, 1988). With these multiple changes, more than 2 crossover events are required for the generation of helper virus, even with the use of gag⁺ vectors such as MFG (Danos and Mulligan, 1988).

In order to create the helper-free producer cell line, it was decided to introduce the pCRIP^{env-} and pCRIP^{gag-2} constructs subsequent to each other, rather than simultaneously, to decrease the risk of recombination and production of replication-competent virus (Danos and Mulligan, 1988). Accordingly, the gag-pol expressing pCRIP^{env-} construct was introduced into 293T/17 cells, and the clone with the highest reverse transcriptase activity was selected. Subsequently, pCRIP^{gag-2}, which expressed the ecotropic envelope, was introduced into the pCRIP^{env-} cell line, and the resulting cell lines were screened for the ability to produce infectious retrovirus upon transfection of the retroviral vector of choice.

### EXAMPLE 3

### CREATION OF ANJOU CELLS:

The first step in creating the helper-free line was to create a cell line expressing high levels of gag-pol. To accomplish this, 6 *µ*g of the pCRIP^{env-} plasmid, linearized with AseI, was transfected into 293T/17 cells together with 3 *µ*g of a plasmid encoding hygromycin resistance as a selectable marker. Individual colonies, grown in hygromycin selection media, were picked and screened for reverse transcriptase activity (Goff et al., 1981). Of 175 clones assayed, 4 clones produced similar or greater amounts of reverse transcriptase than CRE cells; a 3T3-derived producer cell line made by transfecting the pCRIP^{env-} and pCRIP^{gag-2} constructs (Danos and Mulligan, 1988). The best gag-pol producing cell line, #65 (subsequently referred to as ANJOU 65) produced reverse transcriptase at a level 10 times that of CRE and 1/10th that of wild type Moloney virus (Figure 2). The reverse transcriptase activity of ANJOU 65 remained stable for at least 15 passages, even when the cells were grown without hygromycin selection. Transient transfection of ANJOU 65 cells with pBND 8 and the envelope-expressing construct, pCRJP^{gag-2}, gave a retroviral titer of 1.5 x 10⁵ (Table 1).

### EXAMPLE 4

**CREATION OF BOSC 23 CELLS:** The next step in creating the helper-free cell line was to transfect the ecotropic envelope construct and select the cell line producing the highest titer of infectious retrovirus. ANJOU 65 cells were transfected with 10 *µ*g of the AseI linearized ecotropic envelope encoding construct, pCRJP^{gag-2} and 5 *µ*g of the plasmid encoding the selectable marker gene for gpt resistance, gpt2E (Jasin and Berg, 1988). The cells were initially grown in gpt selection media (Mulligan and Berg, 1981); however, once individual clones reached sufficient density to be grown on 10 cm plates, selection was removed and they were screened as described in Materials and Methods. Briefly, clones plated at 2 x 10⁶ cells, 16-24 hours prior to transfection, were transfected with 3 *µ*g pBND. Forty eight hours after transfection, 1 ml of "virus"-containing supernatant was used to infect 3T3 cells, which were assayed for β-galactosidase at 48 hours after infection. Of 70 clones screened, only six produced virus at a titer greater than 10⁴/ml. Of these six, two clones produced virus at titers greater than 10⁵/ml, and one clone, #96 (subsequently referred to as BARTLETT 96), produced virus in excess of 10⁶/ml (1.6 x 10⁶/ml; See Table 1, above).

Because BARTLETT 96 and two subcloned lines all demonstrated a decrease in the ability to package retrovirus over time when grown without gpt selection (Table 1 and data not shown), it appeared that gpt selection could be necessary for maintenance of the high titer phenotype of this subline. To test this hypothesis, passage 2 cells of the BARTLETT 96 line were thawed and cloned by limiting dilution, either in the presence or absence of gpt selection. For these experiments, MFG-lacZ, a β-galactosidase-expressing retroviral vector containing an extended retrovirus RNA packaging site was used in the transfection assay (Spain et al., unpublished). This vector produced infectious retroviral titers approximately 2-fold higher than pBND 8, which does not contain the extended packaging site, following transfection of the BARTLETT 96 cells (data not shown). 24 clones grown in media without selection were analyzed, and 4/24 had viral titers greater than 10⁵/ml and one of these four had a titer greater than 10⁶/ml. 22 clones maintained in gpt selection media were analyzed and 18/22 clones produced virus at a titer greater than 10⁵/ml and 7 of these clones had titers greater than 10⁶/ml. Of these 7 clones, the clone producing the highest titer, BOSC 23, gave a titer of 2.9 x 10⁶/ml (Table 1). BOSC 23, used in all subsequent analyses, was maintained in gpt selective media and its viral titer remained stable for at least 25 passages, thus showing that maintenance of gpt selection is essential for maintaining the high titer phenotype of this clone (Table 1).

### EXAMPLE 5

**CHLOROQUINE TREATMENT OF BOSC23 CELLS DOUBLES THE INFECTIOUS TITER:** In order to maximize the infectious titer, several modifications were made to the CaPO₄ transfection protocol. Growing the BOSC 23 line in 10% FCS with 25 µM chloroquine for the initial 10 hours of transfection resulted in a 2-3 fold increase in titer (Table 1, line 9). This is presumably due to the lysosomal neutralizing activity of the chloroquine (Ausubel et al., 1989). Doubling the volume of all transfection reagents also appeared to result in slightly higher transfection efficiency (Table 1, line 10). The effects of chloroquine and reagent volume on infectious titer appeared to be the result of increasing transfection efficiency since changes in the percent of cells transfected, as measured by FACS, were nearly linear with increases in viral titer. Utilizing these conditions, we have been able to transfect as many as 60% of the BOSC 23 cells, and this has resulted in lacZ infectious titers in the 10⁷/ml range (Table 1, line 10).

### EXAMPLE 6

### TRANSIENT TRANSFECTION OF BOSC 23 PRODUCES HIGH TITER ABL EXPRESSING RETROVIRAL VECTORS:

One reason for creating the BOSC 23 transient retroviral producer system was to obtain high titers of retroviral vectors that express genes that may be unable to be expressed at high titer in stable cell lines. Previous results in this lab have shown that it is very difficult to create either v-*abl* or P210^{*bcr*/*abl*} -helper-free retroviral producing cell lines which maintain G418 titers greater than 10⁵/ml. To test whether our transient system was capable of expressing these genes at a higher titer, either pGD-*v-abl* (Scott et al., 1991) or pGD210^{*bcr*/*abl*} (Daley et al., 1990) was transfected into BOSC 23 cells. Resulting supernatants were then titered by G418 selection.

The parental pGD vector (Daley et al., 1990), containing only a neomycin resistance cassette, produced a viral titer of 3.9 x 10⁶/ml (Table 1). pGD^{*v-abl*} produced virus at a neo titer of 2.9 x 10⁶/ml (Table 1), and the pGD210^{*bcr*/*abl*} produced virus at a neo titer of 1.1 x 10⁶/ml (Table 1). To show that the *abl* gene was transduced as efficiently as the neomycin resistance marker, we infected 3T3s with 1 ml of BOSC 23 produced pGD^{*v-abl*} retrovirus. This transformed almost 100% of the cells (Figure 3A) and is consistent with an infectious titer greater than 10⁶/ml, matching the neo titer.

To demonstrate that the correct *abl* protein product was made by the BOSC 23 produced retroviral vectors, *in vitro abl* kinase assays (Konopka and Witte, 1985) were performed on the infected 3T3 cells. As shown in Figure 3C, the P160 immunoprecipitated from the pGD-v*-abl* infected 3T3s was identical in size to P160^{*v-abl*} from a cell line expressing A-MuLV (N54; (Jackson and Baltimore, 1989). Similarly, P210 was immunoprecipitated from pGD210^{*bcr*/*abl*}-infected 3T3s, and the protein was identical in size to the P210 product of the 210W cell line (Lugo and Witte, 1989), a cell line expressing P210^{*bcr*/*abl*} (Figure 3d). Thus, the BOSC 23 system is readily able to produce both high titers and the correct protein products of retroviral vectors that express toxic genes.

### EXAMPLE 7

**TESTS FOR REPLICATION COMPETENT VIRUS:** To test for the production of helper virus, the BAG indicator cell line was used (Price et al., 1987). This cell line contains integrated β-galactosidase provirus which is capable of being rescued in the presence of either helper virus or retroviral packaging functions. Using this cell line and the following protocol, replication competent virus can be detected at dilutions of at least 10⁻⁶ (data not shown). BAG cells were infected with 1 ml of viral supernatant from pGD (neo titer: 3.9 x 10⁶), pGD^{v-abl} (neo titer: 2.9 x 10⁶), or MFG-tPA (not titered, however MFG-lacZ transfected in parallel had a titer of 7 x 10⁶). Cells were split every 2-3d at 1:10 and 3 mls of supernatant were used to infect 3T3 cells after passage 3, 5, and 10. No β-galactosidase positive cells were observed upon infection of the 3T3s at all 3 time points (9, 15, and 30 rounds of replication, respectively; Table 1, lines 12, 13, 15). As a positive control, MFG-tPA and pZAP were cotransfected and supernatant was used to infect BAG cells. The viral titer in these positive controls was approximately 3 x 10⁴ at all three time points. These results show that under our conditions, the retroviral vectors produced by the BOSC 23 cells are devoid of helper virus.

### EXAMPLE 8

To test the hypothesis that simultaneous introduction of the two plasmids encoding the *gag-pol* and *env* functions would produce replication competent virus, pCRIP^{env-}, pCRIP^{gag-2}, and pBND8 were co-transfected into 293T/17 cells and 48 hours later, 1 ml of supernatant was used to infect BAG cells. The BAG cells were split 1:10 for 3 passages, and then 1 ml of "viral" supernatant was used to infect 3T3s, which were stained for β-galactosidase activity 48 hours after transfection. β-galactosidase positive cells were present indicating that co-introduction of these three elements resulted in the formation of replication competent virus (Table 1, line 2). Thus, in contrast to the present approach using integrated packaging functions, systems that rely on co-introduction by transient transfection of helper packaging functions can result in helper virus formation.

### EXAMPLE 9

### RETROVIRAL VECTORS PRODUCED BY BOSC 23 CELLS ARE CAPABLE OF INFECTING DAY 13 CFU-S:

To determine if retroviral vectors produced by the BOSC 23 system were capable of infecting rare cells in a population, these retroviral vectors were used to infect murine hematopoietic progenitors, as assayed by β-galactosidase activity or proviral integration of d13 CFU-S. Day 13 CFU-S is enriched for hematopoietic stem cells (Spangrude, 1989). 1.25 x 10⁶ Ficoll-banded cells derived from mouse long bones were cocultured in WEHI-3B-conditioned media with BOSC 23 cells which had been transfected 1 day previously with either MFG-lacZ or pGD. Experiments done in parallel showed that the MFG-lacZ transfected cells produced virus at a titer of 5 x 10⁶. 48 hours after cocultivation, the non-adherent cells were pipetted off the adherent later and injected into lethally irradiated recipients. Two mice receiving 900 cGy and 5 x 10⁴ MFG-lacZ infected bone marrow cells survived until day 13, and upon necroscopy, CFU-S were grossly apparent. Four mice receiving 850 cGy, two of which received 5 x 10⁴ pGD-infected bone marrow cells and two of which received 1 x 10⁵ pGD-infected bone marrow cells survived until day 13. CFU-S were not grossly apparent in the spleens of the mice receiving 850 cGy suggesting that this may not have been a lethal irradiation dose. A spleen from one of the mice receiving MFG-lacZ infected bone marrow was stained for β-galactosidase activity (MacGregor et al., 1990), and this spleen was strongly positive, as indicated by two distinct blue areas measuring approximately 0.2 mm in diameter, as well as punctate blue spots throughout the spleen. No β-galactosidase activity was apparent in the 2 spleens stained from the pGD animals (one each from animals receiving 5 x 10⁴ cells and 1 x 10⁵ cells. Cryostat sections were prepared from the pGD and MFG-lacZ spleens, and as shown in Figure 4a, there are two distinct X-gal stained areas in the MFG-lacZ spleen that most likely correspond to CFU-S. In addition, there were individually stained cells in the MFG-lacZ spleen, possibly representing infection of more mature cells. No X-gal stained cells were present in the spleens of the mice receiving pGD-infected bone marrow (Figure 4B).

### EXAMPLE 10

**STRATEGY FOR CREATING THE AMPHOTROPIC HELPER-FREE CELL LINE:** In similar fashion to the preparation of the ecotropic cell line described in Example 2, the preparation of the amphotropic cell line began with the preparation of a helper-free producer cell line from the 293T/17 subline (Figure 1B). The strategy for creating the helper-free producer cell line was generally the same as that of Example 2, and accordingly, a set of plasmids, pCRIP^{env-} and pCRIP^{amgag} were used, which placed the Moloney packaging functions onto 2 plasmids (Danos and Mulligan, 1988). pCRIP^{env-} contains a mutation in the envelope region and expresses both gag and pol products. pCRIP^{amgag} contains mutations in the gag region, and only expresses the amphotropic envelope (Danos and Mulligan, 1988). As with the protocol of Example 2, the retrovirus RNA packaging site was deleted and the 3' LTR was replaced by an SV40 poly A site in both plasmids (Danos and Mulligan, 1988). With these multiple changes, more than 2 crossover events are required for the generation of helper virus, even with the use of gag⁺ vectors such as MFG (Danos and Mulligan, 1988).

In like manner to the preparation of the ecotropic cell line, the pCRIP^{env-} and pCRIP^{amgag} constructs were introduced subsequent to each other, rather than simultaneously, to decrease the risk of recombination and production of replication-competent virus (Danos and Mulligan, 1988). Accordingly, the gag-pol expressing pCRIP^{env-} construct was introduced into 293T/17 cells, and the clone with the highest reverse transcriptase activity was selected. Subsequently, pCRIP^{amgag}, which expressed the amphotropic envelope, was introduced into the pCRIP^{env-} cell line, and the resulting cell lines were screened for the ability to produce infectious retrovirus upon transfection of the retroviral vector of choice.

### EXAMPLE 11

**CREATION OF CAK 8 CELLS:** The next step in creating the amphotropic helper-free cell line was to transfect the amphotropic envelope construct and select the cell line producing the highest titer of infectious retrovirus. ANJOU 65 cells were transfected with 10 *µ*g of the AseI linearized amphotropic envelope encoding construct, pCRIP^{amgag} and 5 *µ*g of the plasmid encoding the selectable marker gene for gpt resistance, gpt2E (Jasin and Berg, 1988). The cells were initially grown in gpt selection media (Mulligan and Berg, 1981); and the population was screened by FACS with an antibody against the amphotropic envelope 83 A25 (B. Cheseboro) in order to select high amphotropic envelope expressing cells. Cells with high envelope expression were sorted as single cells and were grown up. The cell line exhibiting the highest envelope expression was Ampho 18. On growing this cell line in hygromycin medium however, the cells died and in addition, this cell line did not produce infectious retrovirus particles. As a result, the pCRIP^{env-} and pSV2 Hgm constructs were reintroduced. Individual clones were selected and screened by a reverse transcriptase assay. The clone with the highest RT activity was Ampho 133. This clone also produced infectious retroviruses with a lacZ titer of 2 x 10⁶ infectious virus per milliliter on NIH 3T3 cells. Ampho 133 was subcloned and a clone was derived that has been designated CAK 8. CAK 8 is an amphotropic producer cell line that gives lacZ titers of 2 x 10⁶ on NIH 3T3 cells when transfected with 3 µg of MFG-lacZ in the presence of chloroquine.

### DISCUSSION:

As described and confirmed above, a method and corresponding materials for the rapid generation of high titer stocks of helper-free retroviruses have been developed. The invention employs the transient introduction of DNA containing recombinant retroviral DNA sequences and genetic material of interest into mammalian cells. Within three days, transfected cells produce a high titer of the introduced retroviral vector (greater than 10⁶ assayable infectious units/ml), without evidence of helper virus. It is believed that no one has ever made helper-free retroviral stocks of such a titer by transient transfection. The titers obtained in the present transient system are comparable to the best titers obtained utilizing the current producer cell lines and selection for stable clones. Retroviral stocks of high titers are necessary for efficient introduction of recombinant genetic material into rare cells, such as stem cells and infrequently-dividing cells such as neurons, and to be able to infect all cells in a large target population.

The model system employed herein is the highly transfectable 293T cell line, a transformed cell line that expresses the adenovirus E1a gene and SV40 large T antigen. By introducing constructs expressing retroviral packaging functions into a subclone of this cell line, the retroviral packaging cell lines BOSC 23 and CAK 8, and the progenitor lines 293T/17 and ANJOU 65 have been created. BOSC 23 cells are able to produce infectious retroviruses with a titer in the range of 10⁷ infectious particles per ml. Achieving a retroviral titer in the 10⁷ range required several modifications of the typical CaPO₄ transfection protocol. These included growing the cells in media containing 25 *µ*M chloroquine for the initial 10 hours of the transfection, doubling the volumes of all reagents, and optimizing the density of BOSC 23 cells and amount of transfected DNA. These alterations increased the transfectability of and viral production by the BOSC 23 cells, resulting in transient transfection frequencies of approximately 60% of the BOSC 23 cells and higher titer of viruses, respectively.

Compared to the creation of stable producer cell lines, the present system is able to produce virus at high infectious titers, but without the labor, time, and expense involved in selecting and testing individual clones. Selecting high titer producing stable clones requires at least one month of intensive cell culture and testing; whereas the present system allows production of high titer retroviral vectors in less than 72 hours. As a result, the present system greatly facilitates testing of the efficacy of different constructs or different conditions.

A major advantage of both the BOSC 23 and CAK 8 systems is the ability to produce high titers of retroviral vectors which are detrimental to the growth of stable producer cell lines. Our experience with stable producer cell lines expressing either v-*abl*- or P210^{*bcr*/*abl*}- retroviral vectors showed that even when these cell lines initially expressed the retroviral constructs at high titer, the titers fell with continued propagation of the cell line. Although this phenomenon is not well understood, it may be due to epigenetic mechanisms relating to degree of transformation and cellular adherence rather than a direct toxic effect of the gene product (Ziegler et al., 1981). This phenomenon is not limited to retroviral constructs containing members of the *abl* family since a similar phenomenon has been observed with members of the *rel* family of transcription factors (data not shown). Using the BOSC 23 system, it was possible to achieve titers in excess of 10⁶ ml for both v*-abl* and P210^{*bcr*/*abl*} retroviral constructs, and to show that the correct *abl* protein products were produced. Because BOSC 23 cells are exposed to the retroviral construct products for less than 72 hours, any detrimental effects of the product upon the cell line are minimized. The ability of the BOSC 23 system to support high titer retroviral vector production of all genes expressed thus far, including those previously thought to be "toxic", should greatly expand the scope and utility of retroviral vectors.

The BOSC 23 system is also free of detectable replication competent virus. This was demonstrated by a stringent assay in which either packaging of the helper functions or production of helper virus itself would result in the rescue of the integrated lacZ-containing provirus from an indicator cell line (BAG). No rescue was seen utilizing 3 different retroviral vector constructs, including a vector containing an extended retroviral RNA packaging site (MFG-tPA), even though each BAG-infected population underwent at least 30 rounds of replication.

There have been several previous attempts to make high-titer retroviruses by transient transfection. Transient transfection of either the Psi-2 or PA317 cell lines results in titers in the 10⁴/ml range (Price et al., 1987; Miller et al., 1986). Recently, Landau and Littman have described a system in which two retroviral vectors containing SV40 ori sequences and expressing the packaging functions from one vector and the gene of interest from the other vector are cotransfected into COS cells (Landau and Littman, 1992). They have obtained titers as high as 1 x 10⁵, which appear helper-free, and are dependent on the amplification of the SV40 ori-containing vectors. It is somewhat surprising that they do not document production of replication-competent virus since only one homologous recombination event is necessary to create helper virus. Cotransfection of 293T/17 cells with pCRIP^{env-}, pCRIP^{gag-2}, and pBND, which contain the same region of homology as in the Landau system but requires more than 2 recombination events to generate helper virus, results in replication competent virus. If the COS system is free of helper virus, this may suggest that there is a difference in recombination rates between 293 and COS cells.

Because BOSC 23 contains an integrated SV40 large T antigen, it may be possible to increase retroviral titers by using retroviral vectors containing the SV40 origin of replication. In the COS system, titers increased from 10³ to 10⁵ by using vectors containing the SV40 origin of replication (Landau and Littman, 1992). It was attempted to take advantage of this potential amplification by transfecting BARTLETT 96 (and 293T/17 in the presence of helper) with pSPUD, a retroviral vector in which lacZ is driven from the SV40 promoter. However, an amplification effect was not observed, and in fact, the retroviral titers dropped by one log when using this vector (data not shown). One potential drawback to increasing retroviral titer by amplification is that this may increase the recombination rate (Heinzel et al., 1988), and thus, the chance of producing replication competent retrovirus.

The following is a listing of the publications referred to in the foregoing specification.
Anderson, W. F. (1992). Human gene therapy. Science *256,* 808-813.
Ausubel, F. M., Brent, R., Kingston, R. E., Moore, D. D., Seidman, J. G., Smith, J. A., and Struhl, K., eds., *Current Protocols in Molecular Biology,* (John Wiley and Sons, Inc., New York, 1989).
Bender, M. A., Palmer, T. D., Gelinas, R. E., and Miller, A. D. (1987). Evidence that the packaging signal of Moloney murine leukemia virus extends into the gag region. J. Virol. *61*, 1639-1646.
Bernard, H. U., Krammer, G., and Rowekamp, W. G. (1985). Construction of a fusion gene that confers resistance against hygromycin B to mammalian cells in culture. Exp. Cell Res. *158*, 237-243.
Bums, J. C., Friedmann, T., Driever, W., and Burrascano, M. (1993). Vesicular stomatitis virus G glycoprotein pseudotyped retroviral vectors: Concentration to very high titer and efficient gene transfer into mammalian and nonmammalian cells. Proc. Natl. Acad. Sci. USA *90*, 8033-8037.
Daley, G. Q., Van Etten, R. A., and Baltimore, D. (1990). Induction of chronic myelogenous leukemia in mice by the P210 bcr/abl gene of the Philadelphia chromosome. Science *247*, 824-830.
Danos, O., and Mulligan, R. C. (1988). Safe and efficient generation of recombinant retroviruses with amphotropic and ecotropic host ranges. Proc. Natl. Acad. Sci. USA *85,* 6460-6464.
DuBridge, R. B., Tang, P., Hsia, H. C., Phaik-Mooi, L., Miller, J. H., and Calos, M. P. (1987). Analysis of mutation in human cells by using an Epstein-Barr virus shuttle system. Mol. Cell. Biol. *7*, 379-387.
Goff, S., Traktman, P., and Baltimore, D. (1981). Isolation and properties of Moloney murine leukemia virus mutants: use of a rapid assay for release of virion reverse transcriptase. J. Virol. *38,* 239-248.
Graham, F. L., Smiley, J., Russell, W. C., and Naish, R. (1977). Characteristics of a human cell line transformed by DNA from human adenovirus type 5. J. Gen. Virol. *36*, 59-72.
Heinzel, S. S., Krysan, P. J., Calos, M. P., and DuBridge, R. B. (1988). Use of simian virus 40 replication to amplify Epstein-Barr virus shuttle vectors in human cells. J. Virol. *62*, 3738-3746.
Hopkins, N. (1993). Commentary. High titers of retrovirus (vesicular stomatitis virus) pseudotypes, at last. Proc. Natl. Acad. Sci. USA *90,* 8759-8760.
Jackson, P., and Baltimore, D. (1989). N-terminal mutations activate the leukemogenic potential of the myristoylated form of c*-abl.* EMBO J. *8*, 449-456.
Jasin, M., and Berg, P. (1988). Homologous integration in mammalian cells without target gene selection. Genes and Dev. *2*, 1353-1363.
Konopka, J. B., and Witte, O. N. (1985). Detection of c-*abl* tyrosine kinase activity in vitro permits direct comparison of normal and altered *abl* gene products. Mol. Cell. Biol. *5*, 3116-3123.
Landau, N. R., and Littman, D. R. (1992). Packaging system for rapid generation of murine leukemia virus vectors with variable tropism. J. Virol. *66*, 5110-5113.
Lugo, T. G., and Witte, O. N. (1989). The *bcr-abl* oncogene transforms rat-1 cells and cooperates with v*-myc.* Mol. Cell. Biol. *9,* 1263-1270.
MacGregor, G. R., Nolan, G. P., Fiering, S., Roederer, M., and Herzenberg, L. A., in *Methods in Molecular Biology: Gene Transfer and Expression Protocals* E. J. Murray, Eds. (The Humana Press Inc., Clifton, NJ, 1990), pp. 1-19.
Markowitz, D., Goff, S., and Bank, A. (1988). A safe packaging line for gene transfer: separating viral genes on two different plasmids. J. Virol. *62*, 1120-1124.
Miller, A. D. (1990). Retrovirus packaging cells. Human Gene Ther. *1*, 5-14.
Miller, A. D., Trauber, D. R., and Buttimore, C. (1986). Factors involved in production of helper virus-free retrovirus vectors. Som. Cell Mol. Gen. *12*, 175-183.
Mulligan, R. C., and Berg, P. (1981). Selection for animal cells that express the *escherichia coli* gene coding for xanthine-guanine phosphoribosyltransferase. Proc. Natl. Acad. Sci. USA *78*, 2072-2076.
Nolan, G. P., Fiering, S., Nicolas, J. F., and Herzenberg, L. A. (1988). Fluorescence-activated cell analysis and sorting of viable mammalian cells based on β-D-galactosidase activity after transduction of E.col LacZ. Proc. Natl. Acad. Sci. USA *85*, 2603-2607.
Price, J., Turner, D., and Cepko, C. (1987). Lineage analysis in the vertebrate nervous system by retrovirus-mediated gene transfer. Proc. Natl. Acad. Sci. USA *84*, 156-160.
Scott, M. L., Van Etten, R. A., Daley, G. Q., and Baltimore, D. (1991). v-*abl* causes hematopoietic disease distinct from that caused by *bcr-abl.* Proc. Natl. Acad. Sci. USA *88*, 6506-6510.
Shoemaker, C., Hoffmann, J., Goff, S. P., and Baltimore, D. (1981). Intramolecular integration within moloney murine leukemia virus DNA. J. Virol. *40*, 164-172.
Spain, L., Robbins, P., and Mulligan, R. C. unpublished.
Spangrude, G. J. (1989). Enrichment of murine haemopoietic stem cells: diverging roads. Immunol. Today *10*, 344-350.
Turner, D., and Cepko, C. unpublished
Walsh, C., and Cepko, C. (1988). Clonally related cortical cells show several migration patterns. Science *241*, 1342-1345.
Ziegler, S. F., Whitlock, C. A., Goff, S. P., Gifford, A., and Witte, O. N. (1981). Lethal effect of the abelson murine leukemia virus transforming gene product. Cell *27*, 477-486.

This invention may be embodied in other forms or carried out in other ways without departing from the essential characteristics thereof. The present disclosure is therefore to be considered as in all respects illustrative and not restrictive, the scope of the invention being indicated by the appended Claims.

## Claims

1. The clonal cell line 293T/17 having ATCC Accession No. CRL 11268.

2. The clonal cell line ANJOU 65 having ATCC Accession No. CRL 11269.

3. The clonal cell line BOSC 23 having ATCC Accession No. CRL 11270.

4. The clonal cell line CAK 8 having ATCC Accession No. CRL11554.

5. A test kit for the measurement and assessment of the activity of genetic material of interest on a target cellular population, said test kit comprising a cell line of any one of Claims 1 to 4 in combination with reagents for optimal transfection.

## Patentansprüche

1. Klonale Zell-Linie 293T/17, die die ATCC Zugangsnummer CRL 11268 hat.

2. Klonale Zell-Linie ANJOU 65, die die ATCC Zugangsnummer CRL 11269 hat.

3. Klonale Zell-Linie BOSC 23, die die ATCC Zugangsnummer CRL 11270 hat.

4. Klonale Zell-Linie CAK 8, die die ATCC Zugangsnummer CRL 11554 hat.

5. Testkit für die Messung und die Einschätzung der Aktivität von genetischem Material von Interesse auf eine zelluläre Zielpopulation, wobei das Testkit eine Zell-Linie gemäß einem der vorangehenden Ansprüche 1 bis 4 in Kombination mit Reagenzien für eine optimale Transfektion umfaßt.

## Revendications

1. Lignée cellulaire clonale 293T/17 ayant le numéro d'accès ATCC CRL 11268.

2. Lignée cellulaire clonale ANJOU 65 ayant le numéro d'accès ATCC CRL 11269.

3. Lignée cellulaire clonale BOSC 23 ayant le numéro d'accès ATCC CRL 11270.

4. Lignée cellulaire clonale CAK 8 ayant le numéro d'accès ATCC CRL 11554.

5. Kit de test pour la mesure et l'évaluation de l'activité d'un matériau génétique d'intérêt sur une population cellulaire cible, ledit kit comprenant une lignée cellulaire selon l'une quelconque des revendications 1 à 4 en combinaison avec des réactifs pour une transfection optimale.
